# EUROPEAN PATENT APPLICATION

(11) **EP 1 273 287 A1**
(43) Date of publication of application: **08.01.2003**
(21) Application number: 01917589.2
(22) Date of filing: 29.03.2001
(51) Int. Cl.: A61K 9/08, A61K 9/48, A61K 47/14, A61K 31/136, A61K 31/44

(54) **OILY COMPOSITIONS CONTAINING HIGHLY FAT-SOLUBLE DRUGS**

(30) Priority: 04.04.2000 JP 2000102272
(71) Applicant: SHIONOGI & CO., LTD., Osaka-shi, Osaka 541-0045 (JP)
(72) Inventor: NISHIHARA, Yoshitaka, c/o Shionogi & Co. Ltd., Osaka-shi, Osaka 553-0002 (JP); KINOSHITA, Haruki, c/o Shionogi & Co. Ltd., Osaka-shi, Osaka 553-0002 (JP); YOSHIKAWA, Takayoshi, c/o Shionogi & Co. Ltd., Osaka-shi, Osaka 553-0002 (JP)
(74) Representative: VOSSIUS & PARTNER
(86) International application number: JP0102621
(87) International publication number: WO01074331

(57) **Abstract**

The present invention provides an oil preparation comprising a high lipophilic medicament such as a compound of the formula (I): 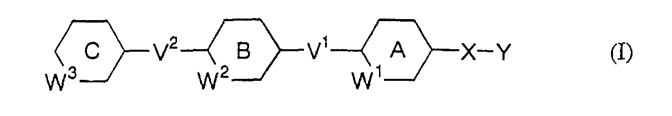 wherein the ring A, ring B and ring C are each independently optionally substituted aromatic carbocycle, optionally substituted heterocycle or the like,
W¹, W² and/or W³ represents a single bond when the ring A, ring B and/or ring C is optionally substituted 5-membered heterocycle,
X is a single bond, -O-, -CH₂-, -NH- or the like,
Y is lower alkyl, lower alkenyl or the like
   or a pharmaceutically acceptable salt or solvate thereof as an active ingredient, (i) a medium-chain triglyceride and/or a propylene glycol medium-chain fatty acid ester, (ii) a long-chain triglyceride and (iii) a surfactant.

## Description

### Technical Field

The present invention relates to oil preparations improving bioavailability (hereinafter referred to as BA).

### Background Art

Though tricyclic compounds used as an active ingredient in the present preparations are disclosed in WO97/39999, WO98/04508 and WO99/38829 the present oil preparations are not disclosed.

In JP 90/255623-A, a pharmaceutical preparation comprising (a) cyclosporin as active ingredient, (b) a triglyceride, (c) partial ester of glycerol with fatty acid, or complete or partial ester of propylene glycol or sorbitol, and (d) a surfactant are disclosed. Any combined effect of a medium-chain triglyceride and a long-chain triglyceride, however, is not explained.

Oral oil preparations comprising a low water-solubility drug (high lipophilic drug), a medium-chain triglyceride having 8 to 12 carbon atoms and lecithin are shown in JP 96/92088 A. Long-chain triglycerides such as soybean oil and sesame oil are described as inappropriate additives.

A suspension or solution of esters or amides in a medium-chain glyceride is disclosed in JP 89/25715 A. Lecithin is exemplified as a viscosity reducing agent. Long-chain triglycerides are not mentioned as additives.

The low solubility of high lipophilic medicament in gastrointestinal tracts is one of the reasons of low BA and various methods to solve this problem have been examined. For example, in Griseofulvin or Nifedipine preparations, crystals of medicaments are finely crushed to improve BA. Tacrolimus preparation is manufactured by using the medicament in a form of amorphous to improve the dissolution rate and BA.

### Disclosure of Invention

The object of the present invention is to provide oil preparations having superior BA.

The present invention provides
[1] An oil preparation comprising high lipophilic medicament as an active ingredient, (i) a medium-chain triglyceride (hereinafter referred to as MCT) and/or a propylene glycol medium-chain fatty acid ester (hereinafter referred to as PG), (ii) a long-chain triglyceride (hereinafter referred to as LCT) and (iii) a surfactant,
[2] The oil preparation as described in [1], wherein the active ingredient is a compound of the formula (I): 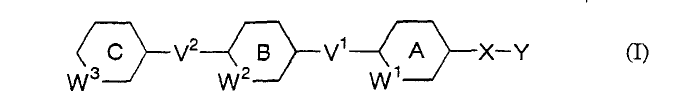
   wherein the ring A, ring B and ring C are each independently optionally substituted aromatic carbocycle or optionally substituted 5- or 6-membered heterocycle which may fuse with benzene ring,
   W¹, W² and/or W³ represents a single bond when the ring A, ring B and/or ring C is optionally substituted 5-membered heterocycle,
   X is a single bond, -O-, -CH₂-, -NR¹- wherein R¹ is hydrogen, optionally substituted lower alkyl, lower alkenyl or lower alkylcarbonyl, or -S(O)p- wherein p is an integer of 0 to 2,
   Y is hydrogen, optionally substituted lower alkyl, optionally substituted lower alkoxy, optionally substituted lower alkenyl, optionally substituted lower alkynyl, optionally substituted acyl, optionally substituted cycloalkyl, optionally substituted cycloalkenyl, optionally substituted lower alkoxycarbonyl, optionally substituted sulfamoyl, optionally substituted amino, optionally substituted aryl or optionally substituted 5- or 6-membered heterocycle,
   R¹ taken together with Y may form -(CH₂)m-, -(CH₂)₂-Q-(CH₂)₂- (wherein Q is CH₂, O, S or NR'), -CR'=CH-CH=CR'-, -CH=N-CH=CH-, -N=CH-N=CH-, -C(=O)-O(CH₂)r-, -C(=O)-NR'-(CH₂)r- or -C(=O)-NR'-N=CH- wherein m is 4 or 5, r is 2 or 3, R' is hydrogen, lower alkyl or lower alkenyl,
   Y may be halogen when X is -CH₂- or -NR¹-,
   Y may be optionally substituted lower alkylsulfonyl or optionally substituted arylsulfonyl when X is -O- or -NR¹-,
   one of V¹ and V² is a single bond and the other is a single bond, -O-, -NH-, -OCH₂-, -CH₂O-, -CH=CH-, -C≡C-, -CH(OR²)- (wherein R² is hydrogen or lower alkyl), -CO-, -NHCHR³- or-CHR³NH- wherein R³ is hydrogen or hydroxy,
      or a pharmaceutically acceptable salt or solvate thereof as an active ingredient,
[3] The oil preparation as described in [1], wherein the medium-chain triglyceride and/or the propylene glycol medium-chain fatty acid ester is a mixture containing triglyceride with caprylic acid and triglyceride with capric acid (for example, ODO),
[4] The oil preparation as described in any one of [1] to [3] wherein the long-chain triglyceride is vegetable oil,
[5] The oil preparation as described in any one of [1] to [4] wherein the surfactant is lecithin,
[6] The oil preparation as described in any one of [1] to [5] wherein the active ingredient is 0.1 to 20 % by weight, the total amount of (i) a medium-chain triglyceride and/or a propylene glycol medium-chain fatty acid ester and (ii) a long-chain triglyceride is 60 to 99 % by weight and (iii) a surfactant is 0.1 to 20 % by weight, each percentage being to the total amount of the oil preparation,
[7] The oil preparation as described in any one of [1] to [5] wherein the active ingredient is 1 to 10 % by weight, the total amount of (i) a medium-chain triglyceride and/or a propylene glycol medium-chain fatty acid ester and (ii) a long-chain triglyceride is 80 to 95 % by weight and (iii) a surfactant is 1 to 10 % by weight, each percentage being to the total amount of the oil preparation,
[8] The oil preparation as described in any one of [1] to [7], wherein the weight ratio of (i) a medium-chain triglyceride and/or a propylene glycol medium-chain fatty acid ester and (ii) a long-chain triglyceride is in a range of 97:3 to 10:90,
[9] The oil preparation as described in any one of [1] to [7], wherein the weight ratio of (i) a medium-chain triglyceride and/or a propylene glycol medium-chain fatty acid ester and (ii) a long-chain triglyceride is in the range of 70:30 to 30:70,
[10] The oil preparation as described in any one of [1] to [9] wherein the active ingredient is a compound of the formula (Ia): 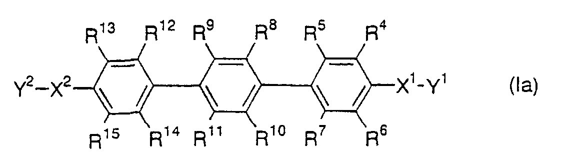
   wherein R⁴, R⁵, R⁶, R⁷, R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, carboxy or lower alkoxycarbonyl,
   X¹ and X² are each independently -O-, -CH₂- or -NH-,
   Y¹ and Y² are each independently optionally substituted lower alkyl, optionally substituted aryl(lower)alkyl or optionally substituted lower alkenyl,
      or a pharmaceutically acceptable salt or solvate thereof,
[11] The oil preparation as described in any one of [1] to [9] wherein the active ingredient is a compound of the formula (Ib): 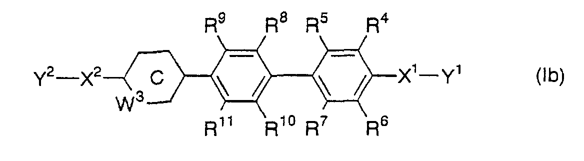 wherein the ring C is optionally substituted 5- or 6-membered heterocycle which contains one or two hetero atoms, W³ represents a single bond when the ring C is a 5-membered heterocycle and the other symbols are the same as defined in [9],
   or pharmaceutically acceptable salt or solvate thereof,
[12] A pharmaceutical preparation comprising the oil preparation according to any one of [1] to [11],
[13] The pharmaceutical preparation as described in [12] which is a soft capsule preparation.

### Brief Description of the Drawings

Figure 1 shows concentrations of Compound (I-1) in plasma when the present preparations or reference preparations were administered to dogs.
Figure 2 shows concentrations of Compound (I-2) in plasma when the present preparation or a reference preparation (long-chain triglycerides additive-free) was administered to rats.
Figure 3 shows concentrations of Compound (I-2) in plasma when a reference preparation or the present preparations using various kinds of long-chain triglycerides were administered to rats.
Figure 4 shows concentrations of Compound (I-1) in plasma when reference preparations or the present preparations using various ratio of medium-chain triglycerides and long-chain triglycerides were administered to rats.
Figure 5 shows the correlation between oral absorption and the ratio of medium-chain triglycerides and long-chain triglycerides.
Figure 6 shows concentrations of Compound (I-2) in plasma when reference preparations or the present preparations were administered to rats.
Figure 7 shows concentrations of Compound (I-1) in plasma when reference preparations or the present preparations using various kinds and the concentrations of surfactants were administered to rats.

### Best Mode for Carrying Out the Invention

In the present oil preparation, any high lipophilic medicament can be preferably used as an active ingredient. "High lipophilic medicament" includes medicaments whose Octanol/Water Partition Coefficient (log P) is 4 or more, preferably 5 or more.

Octanol/Water Partition Coefficient can be calculated by the following method, for example.

A proper amount of medicament for calculating the partition coefficient is mixed with a proper amount of octanol and water, then shaked to make equilibrium. Each concentrations of the medicament in octanol layer and aqueous layer is measured by HPLC and the partition ratio is calculated.

Examples of "high lipophilic medicament" are the compounds of the above formula (I), cyclosporin, nifedipine, griseofulvin, menatetrenone, α-tocopherol, L-aspargic acid, dequalinium chloride, estradiol, anethole, trithione, allylestrenol, amiodarone, amitriptyline, bepridil, bromocriptine, bupivacaine, chloroquine, chlorpromazine, clemastine, clozapine, desipramine, diflunisal, doxazosin, felodipine, flecainide, fluoxetine, flurazepam, flurbiprofen, fosinopril, glyburide, imipramine, indomethacin, isotretinoin, isradipine, itraconazole, ketoconazole, lorcainide, lovastatin, nicardipine, nimodipine, nitrendipine, nortriptyline, pentazocine, protriptyline, sertraline, simvastatin, tacrolimus, tamoxifen, terfenadine, trazodone, prazepam and loratadine, preferably the following compounds of the formula (I), pharmaceutically acceptable salt or solvate thereof.
1) The compound wherein ring A is optionally substituted benzene, optionally substituted pyridine, optionally substituted pyrimidine, optionally substituted pyridazine, optionally substituted pyrazine wherein the substituents are halogen, hydroxy, lower alkyl, lower alkoxy, carboxy or lower alkoxycarbonyl (hereinafter referred to as "ring A is A1"),
   the compound wherein ring A is optionally substituted benzene, optionally substituted pyridine or optionally substituted pyrimidine wherein the substituents are halogen, hydroxy or lower alkoxy (hereinafter referred to as "ring A is A2"),
   the compound wherein ring A is optionally substituted pyridine or optionally substituted benzene wherein the substituents are halogen (hereinafter referred to as "ring A is A3"),
   the compound wherein ring A is benzene optionally substituted with halogen (hereinafter referred to as "ring A is A4"),
   the compound wherein ring A is pyridine optionally substituted with halogen (hereinafter referred to as "ring A is A5"),
2) the compound wherein ring B is optionally substituted benzene wherein the substituents are halogen, hydroxy, optionally substituted lower alkyl, optionally substituted lower alkoxy or optionally substituted lower alkoxycarbonyl (hereinafter referred to as "ring B is B1"),
   the compound wherein ring B is optionally substituted benzene wherein the substituents are halogen, hydroxy, lower alkyl, lower alkoxy or lower alkoxycarbonyl (hereinafter referred to as "ring B is B2"),
   the compound wherein ring B is 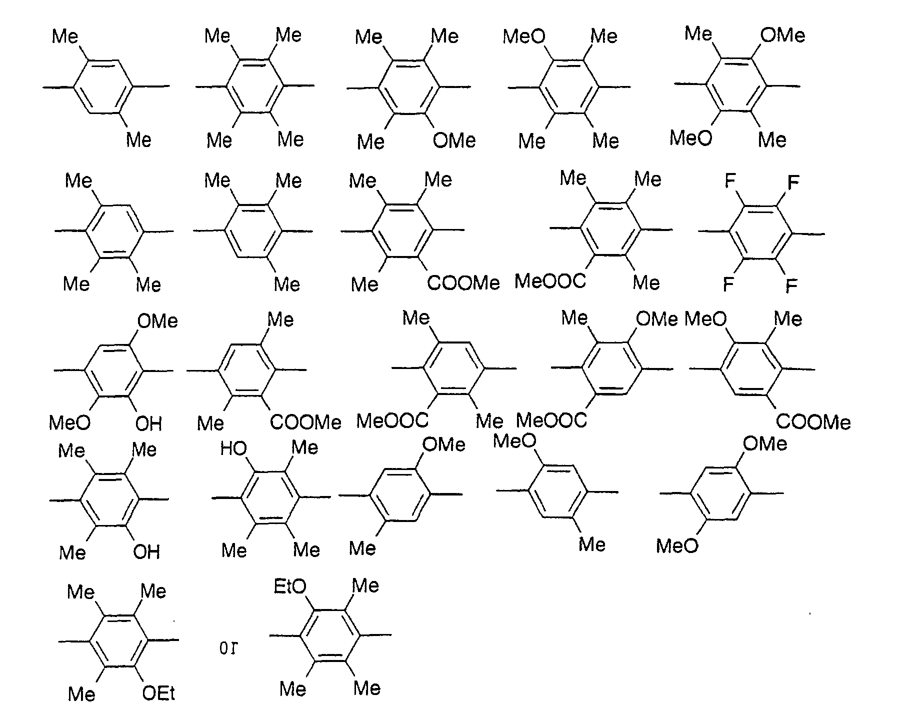 (hereinafter referred to as "ring B is B3"),
3) the compound wherein ring C is optionally substituted benzene, optionally substituted pyridine, optionally substituted pyrimidine, optionally substituted pyridazine, optionally substituted pyrazine wherein the substituents are halogen, lower alkyl, lower alkoxy, lower alkenyloxy, lower alkylamino, cycloalkyl(lower)alkylamino and/or lower alkenylamino (hereinafter referred to as "ring C is C1"),
   the compound wherein ring C is optionally substituted benzene or optionally substituted pyridine wherein the substituents are halogen, lower alkyl, lower alkoxy, prenyloxy, lower alkylamino, cycloalkyl(lower)alkylamino and/or prenylamino (hereinafter referred to as "ring C is C2"),
4) the compound wherein X is -O- or -NH- (hereinafter referred to as "X is X1"),
   the compound wherein X is -NH- (hereinafter referred to as "X is X2"),
5) the compound wherein Y is lower alkyl, cycloalkyl(lower)alkyl, benzyl or lower alkenyl (hereinafter referred to as "Y is Y1"),
   the compound wherein Y is prenyl (hereinafter referred to as "Y is Y2"),
6) the compound wherein both of V¹ and V² are single bonds,
7) the compound wherein a combination of ring A, ring B, ring C, X and Y is any one of the followings and both of V¹ and V² are single bonds,
   (A1, B1, C1, X1, Y1), (A1, B1, C1, X1, Y2), (A1, B1, C1, X2, Y1), (A1, B1, C1, X2, Y2), (A1, B1, C2, X1, Y1), (A1, B1, C2, X1, Y2), (A1, B1, C2, X2, Y1), (A1, B1, C2, X2, Y2), (A1, B2, C1, X1, Y1), (A1, B2, C1, X1, Y2), (A1, B2, C1, X2, Y1), (A1, B2, C1, X2, Y2), (A1, B2, C2, X1, Y1), (A1, B2, C2, X1, Y2), (A1, B2, C2, X2, Y1), (A1, B2, C2, X2, Y2), (A1, B3, C1, X1, Y1), (A1, B3, C1, X1, Y2), (A1, B3, C1, X2, Y1), (A1, B3, C1, X2, Y2), (A1, B3, C2, X1, Y1), (A1, B3, C2, X1, Y2), (A1, B3, C2, X2, Y1), (A1, B3, C2, X2, Y2), (A2, B1, C1, X1, Y1), (A2, B1, C1, X1, Y2), (A2, B1, C1, X2, Y1), (A2, B1, C1, X2, Y2), (A2, B1, C2, X1, Y1), (A2, B1, C2, X1, Y2), (A2, B1, C2, X2, Y1), (A2, B1, C2, X2, Y2), (A2, B2, C1, X1, Y1), (A2, B2, C1, X1, Y2), (A2, B2, C1, X2, Y1), (A2, B2, C1, X2, Y2), (A2, B2, C2, X1, Y1), (A2, B2, C2, X1, Y2), (A2, B2, C2, X2, Y1), (A2, B2, C2, X2, Y2), (A2, B3, C1, X1, Y1), (A2, B3, C1, X1, Y2), (A2, B3, C1, X2, Y1), (A2, B3, C1, X2, Y2), (A2, B3, C2, X1, Y1), (A2, B3, C2, X1, Y2), (A2, B3, C2, X2, Y1), (A2, B3, C2, X2, Y2), (A3, B1, C1, X1, Y1), (A3, B1, C1, X1, Y2), (A3, B1, C1, X2, Y1), (A3, B1, C1, X2, Y2), (A3, B1, C2, X1, Y1), (A3, B1, C2, X1, Y2), (A3, B1, C2, X2, Y1), (A3, B1, C2, X2, Y2), (A3, B2, C1, X1, Y1), (A3, B2, C1, X1, Y2), (A3, B2, C1, X2, Y1), (A3, B2, C1, X2, Y2), (A3, B2, C2, X1, Y1), (A3, B2, C2, X1, Y2), (A3, B2, C2, X2, Y1), (A3, B2, C2, X2, Y2), (A3, B3, C1, X1, Y1), (A3, B3, C1, X1, Y2), (A3, B3, C1, X2, Y1), (A3, B3, C1, X2, Y2), (A3, B3, C2, X1, Y1), (A3, B3, C2, X1, Y2), (A3, B3, C2, X2, Y1), (A3, B3, C2, X2, Y2), (A4, B3, C2, X2, Y1), (A5, B3, C2, X2, Y1).
8) the compound of the formula (Ia'): 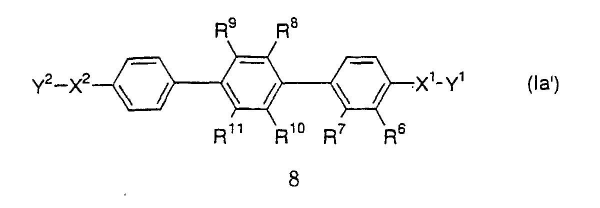 wherein one of R⁶ and R⁷ is hydrogen and the other is halogen, R⁸, R⁹, R¹⁰ and R¹¹ are each independently hydrogen, hydroxy, lower alkyl, lower alkoxy or lower alkoxycarbonyl and the other symbols are the same as defined in [10],
9) the compound of the formula (Ib'): 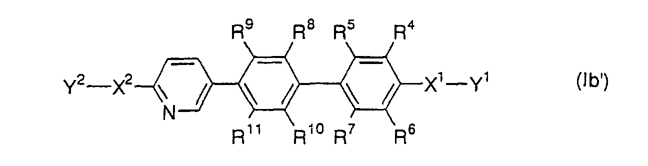 wherein R⁴ and R⁵ are each independently hydrogen, halogen or lower alkoxy, R⁶ and R⁷ are each independently hydrogen, halogen or lower alkyl, R⁸ and R¹¹ are simultaneously lower alkyl or one of R⁸ and R¹¹ is lower alkyl and the other is lower alkoxy or lower alkoxycarbonyl, R⁹ and R¹⁰ are simultaneously hydrogen or lower alkyl, -X¹-Y¹ and -X²-Y² are each independently optionally substituted lower alkylamino, optionally substituted lower alkenylamino, optionally substituted lower alkoxy or optionally substituted lower alkenyloxy,
10) the compound of the above formula (Ib') wherein one of -X¹-Y¹ and -X²-Y² is optionally substituted lower alkylamino or optionally substituted lower alkenylamino and the other is prenyloxy,
11) the compound of the above formula (Ib') wherein 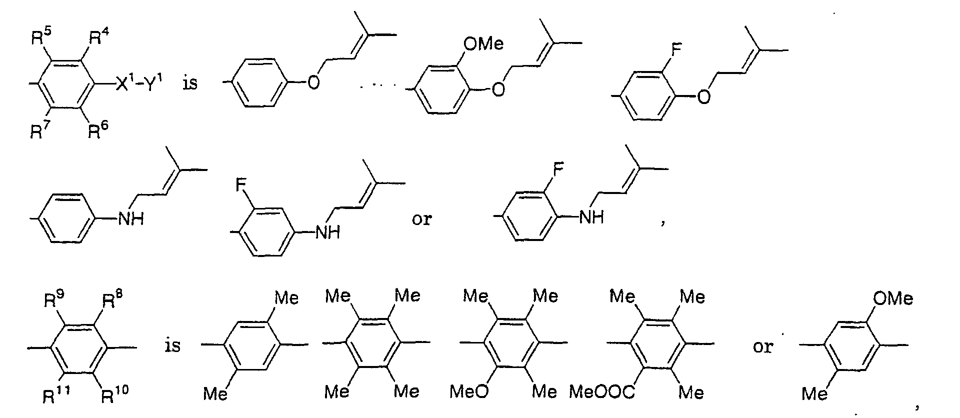 -X²-Y² is optionally substituted lower alkylamino or optionally substituted lower alkenylamino when -X¹-Y¹ is prenyloxy, -X²-Y² is optionally substituted lower alkoxy or optionally substituted lower alkenyloxy when -X¹-Y¹ is prenylamino,
   and
12) the compound of the formula (Ic):

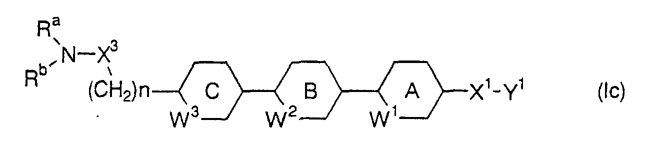

wherein ring A, ring B and ring C is each independently optionally substituted benzene or optionally substituted 5- or 6-membered heterocycle which contains one or two hetero atoms, W¹, W² and/or W³ represents a single bond when the ring A, ring B and/or ring C is optionally substituted 5-membered heterocycle,
X¹ and Y¹ are the same as defined in [10],
X³ is -O- or -NH-,
R^{a} and R^{b} are each independently hydrogen, optionally substituted lower alkyl, optionally substituted lower alkenyl, optionally substituted aryl, optionally substituted cycloalkyl, optionally substituted acyl or optionally substituted lower alkoxycarbonyl or R^{a} and R^{b} taken together may form R^{c}R^{d}C= or-(CR^{e}R^{f})s-,
R^{c} and R^{d} are each independently hydrogen, optionally substituted lower alkyl, optionally substituted lower alkenyl, optionally substituted lower alkynyl, optionally substituted lower alkoxy, optionally substituted lower alkylthio, optionally substituted lower alkenyloxy, optionally substituted lower alkynyloxy, optionally substituted cycloalkyl, optionally substituted aryl or optionally substituted 5- or 6-membered heterocycle or
R^{c} and R^{d} are taken together with a carbon atom to which R^{c} and R^{d} are attached may form cycloalkylidene,
R^{e} are each independently hydrogen, lower alkyl, lower alkoxy or amino,
R^{f} are each independently hydrogen, lower alkyl, lower alkoxy or amino,
n is an integer of 0 to 2 and s is an integer of 2 to 6.

In the present specification, the term "halogen" includes fluorine, chlorine, bromine and iodine, preferably fluorine or chlorine.

The term "lower alkyl" includes C1 to C10, preferably C1 to C8, more preferably C1 to C6 and most preferably C1 to C3 straight or branched alkyl. The examples of "lower alkyl" are methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.

The examples of substituents for "optionally substituted lower alkyl" are (i) halogen; (ii) hydroxy; (iii) lower alkoxy optionally substituted with lower alkoxy; (iv) acyl; (v) acyloxy; (vi) carboxy; (vii) lower alkoxycarbonyl; (viii) mercapto; (ix) lower alkylthio;
(x) amino optionally substituted with hydroxy, lower alkyl or optionally substituted acyl; (xi) imino optionally substituted with
   hydroxy,
   lower alkoxy,
   carboxy(lower)alkoxy,
   aryl(lower)alkoxy or
   5- or 6-membered heterocycle;
(xii) hydrazono optionally substituted with carbamoyl or lower alkoxycarbonyl;
(xiii) hydrazino optionally substituted with
   lower alkyl,
   lower alkenyl,
   optionally substituted lower alkylidene or
   cycloalkylidene;
(xiv) aminooxy optionally substituted with
   lower alkyl,
   lower alkenyl,
   optionally substituted lower alkylidene or
   cycloalkylidene;
(xv) carbamoyl optionally substituted with lower alkyl or amino; (xvi) thiocarbamoyl optionally substituted with lower alkyl; (xvii) cycloalkyl optionally substituted with lower alkyl or lower alkoxy; (xviii) cycloalkenyl optionally substituted with lower alkyl; (xix) cyano;
(xx) phenyl optionally substituted with
   hydroxy,
   lower alkyl,
   carboxy,
   lower alkoxycarbonyl or
   lower alkoxy;
or (xxi) 5- or 6-membered heterocycle which may fuse with a benzene ring and which may be substituted with lower alkyl. "Optionally substituted lower alkyl" may be substituted with these substituents at any possible positions. Preferable examples of substituents are (i) halogen; (ii) hydroxy; (iii) acyloxy; (iv) phenyl optionally substituted with lower alkyl or lower alkoxy; or (v) pyridyl.

The lower alkyl part in "lower alkoxy" is the same as defined in the above "lower alkyl".

The examples of substituents for "optionally substituted lower alkoxy" are halogen; hydroxy; lower alkoxy; acyl; acyloxy; carboxy; lower alkoxycarbonyl; lower alkylthio; amino optionally substituted with lower alkyl; phenyl optionally substituted with lower alkyl or lower alkoxy; heterocycle; heterocyclylcarbonyloxy.

The lower alkyl parts in "lower alkoxycarbonyl", "lower alkylsulfonyl", "lower alkylsulfonyloxy", "lower alkylthio", "lower alkylamino", "cycloalkyl(lower)alkylamino", "aryl(lower)alkyl" and "lower alkylenedioxy" are the same as defined in the above "lower alkyl". The substituents for "optionally substituted lower alkoxycarbonyl", "optionally substituted lower alkylsulfonyl" and "optionally substituted lower alkylthio" are the same as those for the above "optionally substituted lower alkoxy".

The term "lower alkylidene" includes C1 to C10, preferably C1 to C6 and more preferably C1 to C3 divalent hydrocarbon group. Examples are methylidene, ethylidene, propylidene, isopropylidene, butylidene, pentylidene, hexylidene, heptylidene, octylidene, nonylidene and decylidene.

Examples of substituents for "optionally substituted lower alkylidene" are optionally substituted lower alkenyl, optionally substituted lower alkoxy, optionally substituted lower alkylthio, optionally substituted cycloalkyl, optionally substituted aryl or optionally substituted 5- or 6-membered heterocycle, preferably lower alkenyl, lower alkoxy, cycloalkyl, phenyl or 5- or 6-membered heterocycle.

The term "lower alkenyl" includes C2 to C10, preferably C2 to C8 and more preferably C3 to C6 straight or branched alkenyl having one or more double bonds at any possible positions. Examples are vinyl, propenyl(2-propenyl etc.), isopropenyl, butenyl, isobutenyl, prenyl, butadienyl, pentenyl, isopentenyl, pentadienyl, hexenyl, isohexenyl, hexadienyl, heptenyl, octenyl, nonenyl and decenyl. The substituents for "optionally substituted lower alkenyl" are the same as those for the above "optionally substituted lower alkoxy". The halogenated or unsubstituted lower alkenyl are preferable.

The lower alkenyl parts in "lower alkenyloxy", "lower alkenyloxycarbonyl" and "lower alkenylamino" are the same as the above "lower alkenyl". The substituents for "optionally substituted lower alkenyloxy" are the same as those for the above "optionally substituted lower alkoxy".

The term "lower alkynyl" includes C2 to C10, preferably C2 to C8 and more preferably C3 to C6 straight or branched alkynyl. Examples are ethynyl, propynyl (2-propynyl etc.), butynyl (2-butynyl etc.), pentynyl, hexynyl, heptynyl, octynyl, nonynyl and decynyl. These have one or more triple bonds at any possible positions and may have double bonds. The substituents for "optionally substituted lower alkynyl" are the same as those for the above "optionally substituted lower alkoxy".

The term "acyl" includes (1) C1 to C20, preferably C1 to C15, preferably C1 to C8, more preferably C1 to C6 and more preferably C1 to C4 straight or branched chain aliphatic acyl, (2) C4 to C9 and preferably C4 to C7 cyclic aliphatic acyl and (3) aroyl. Examples are formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl and benzoyl.

The term "aroyl" includes aromatic acyl which is formed by excluding one hydroxy from aromatic carboxylic acid.

The substituents for "optionally substituted acyl" are the same as the above "optionally substituted lower alkoxy". Cyclic aliphatic acyl and aroyl may be substituted with lower alkyl. The preferable substituents are halogen.

The acyl part in "acyloxy" is the same as the above "acyl".

The term "lower alkylcarbonyl" includes C2 to C4 aliphatic acyl and examples are acetyl, propionyl, butylyl and isobutylyl. Acetyl is preferable.

The term "cycloalkyl" includes C3 to C6 carbocycle and the examples are cyclopropyl, cyclobutyl, cyclopentyl and cyclohexyl. Substituents for "optionally substituted cycloalkyl" are exemplified by lower alkyl, halogen, hydroxy, carboxy, lower alkoxycarbonyl, lower alkoxy, lower alkylenedioxy, imino optionally substituted with lower alkoxy, aryl and 5- or 6-membered heterocycle. "Optionally substituted cycloalkyl" may be substituted with these substituents at any possible positions.

The cycloalkyl part in "cycloalkyl(lower)alkylamino" is the same as the above "cycloalkyl".

The term "cycloalkenyl" includes the group having at least one double bond at any possible positions in the above "cycloalkyl" and is exemplified by cyclopropenyl, cyclobutenyl, cyclopentenyl, cyclohexenyl and cyclohexadienyl. Substituents for "optionally substituted cycloalkenyl" are the same as those for the above "cycloalkyl".

The term "cycloalkylidene" includes C3 to C6 divalent carbocyclic group and exemplified by cyclopropylidene, cyclobutylidene, cyclopentylidene and cyclohexyliden. The substituents for "optionally substituted cycloalkylidene" are the same as those for the above "cycloalkyl". Unsubstituted cycloalkylidene is preferable.

Examples of substituents for "optionally substituted amino" are
(i) optionally substituted lower alkyl wherein the substituents are
   lower alkoxy,
   cycloalkyl,
   optionally substituted amino wherein the substituents are aroyl optionally substituted with acyloxy(lower)alkoxy,
   optionally substituted aryl wherein the substituents are lower alkyl, lower alkoxy, carboxy or lower alkoxycarbonyl or
   heterocycle;
(ii) lower alkenyl; (iii) lower alkynyl; (iv) cycloalkyl; (v) aryl optionally substituted with lower alkyl, carboxy, acyl or lower alkoxycarbonyl; (vi) sulfamoyl optionally substituted with lower alkyl; (vii) lower alkoxycarbonyl (wherein the substituents are halogen or heterocyclylcarbonyloxy); and (viii) lower alkylsulfonyl.

The term "optionally substituted sulfamoyl" includes sulfamoyl which may be substituted with lower alkyl, lower alkenyl, lower alkynyl or the like.

The term "aromatic carbocyclyl" includes monocyclic or polycyclic aromatic carbocyclyl and is exemplified by benzene, naphthalene, anthracene, phenanthrene and indene, preferably benzene.

The term "aryl" includes groups which are formed by excluding one hydrogen from monocyclic or polycyclic aromatic carbocyclic group and is exemplified by phenyl, naphthyl, anthryl, phenanthryl and indenyl, preferably phenyl.

Examples of substituents for "optionally substituted aromatic carbocycle" and "optionally substituted aryl" are (i) halogen; (ii) hydroxy; (iii) lower alkyl optionally substituted with halogen or carboxy; (iv) lower alkoxy optionally substituted with halogen, aryl, heteroaryl or lower alkoxy; (v) lower alkenyl; (vi) lower alkynyl; (vii) cycloalkyl; (viii) lower alkenyloxy; (ix) lower alkynyloxy; (x) cycloalkoxy; (xi) acyl; (xii) acyloxy; (xiii) carboxy; (xiv) lower alkoxycarbonyl; (xv) lower alkenyloxycarbonyl; (xvi) lower alkylthio; (xvii) lower alkynylthio;
(xviii) amino optionally substituted with
   lower alkyl,
   cycloalkyl(lower)alkyl,
   aryl(lower)alkyl,
   heteroaryl(lower)alkyl,
   lower alkenyl,
   cycloalkyl,
   acyl optionally substituted with halogen,
   lower alkoxycarbonyl or
   lower alkylsulfonyl;
(xix) hydrazino optionally substituted with
   lower alkyl,
   lower alkenyl,
   optionally substituted lower alkylidene or
   cycloalkylidene;
(xx) aminooxy optionally substituted with
   lower alkyl,
   lower alkenyl,
   optionally substituted lower alkylidene or
   cycloalkylidene;
(xxi) guanidino; (xxii) nitro; (xxiii)lower alkylsulfonyl; (xxiv) dihydroxyborane; (xxv) lower alkylsulfonyloxy optionally substituted with halogen; (xxvi) arylsulfonyl; (xxvii) arylsulfonyloxy; (xviii) aryl or (xxix) 5- or 6-membered heterocyclyl. "Optionally substituted aromatic carbocycle" may be substituted with these substituents at any possible positions. The preferable substituents are (i) halogen; (ii) hydroxy; (iii) lower alkyl optionally substituted with halogen; (iv)lower alkoxy optionally substituted with aryl or lower alkoxy; (v) lower alkenyloxy; (vi) acyloxy; (vii) lower alkylthio;
(viii) amino optionally substituted with
   lower alkyl,
   lower alkenyl,
   acyl optionally substituted with halogen or
   lower alkylsulfonyl;
(ix) nitro; (x) lower alkylsulfonyl; (xi) lower alkylsulfonyloxy optionally substituted with halogen; or (xii) arylsulfonyloxy.

The aryl parts in "arylsulfonyl", "arylsulfonyloxy" and "aryl(lower)alkyl" are the same as the above "aryl" and phenyl is preferable. The substituents for "optionally substituted arylsulfonyl" and "optionally substituted aryl(lower)alkyl" are the same as the above "optionally substituted aryl". Unsubstituted arylsulfonyl and unsubstituted aryl(lower)alkyl are preferable.

The term "5- or 6-membered heterocycle" includes 5- or 6-membered heterocycle which contains at least one of hetero atoms arbitrarily selected from a group of O, S and N. Examples of heterocycle are aromatic heterocycle such as pyrrole ring, imidazole ring, pyrazole ring, pyridine ring, pyridazine ring, pyrimidine ring, pyrazine ring, triazole ring, triazine ring, isoxazole ring, oxazole ring, oxadiazole ring, isothiazole ring, thiazole ring, thiadiazole ring, furan ring and thiophene ring, aliphatic heterocycle such as tetrahydropyran ring, dihydropyridine ring, dihydropyridazine ring, dihydropyrazine ring, dioxane ring, oxathiolane ring, thiane ring, pyrrolidine ring, pyrroline ring, imidazolidine ring, imidazoline ring, pyrazolidine ring, pyrazoline ring, piperidine ring, piperazine ring and morpholine ring.

The preferable examples of "5- or 6-membered heterocycle" for ring A, ring B and ring C are 2,5-pyridinediyl and 2,5-pyrimidinediyl.

The term "5- or 6-membered heterocycle which contains one or two hetero atoms" includes aromatic heterocycle such as pyrrole ring, imidazole ring, pyrazole ring, pyridine ring, pyridazine ring, pyrimidine ring, pyrazine ring, isoxazole ring, oxazole ring, isothiazole ring, thiazole ring, furan ring and thiophene ring and aliphatic heterocycle such as dioxane ring, oxathiolane ring, thiane ring, dihydropyridine ring, pyrrolidine ring, pyrroline ring, imidazolidine ring, imidazoline ring, pyrazolidine ring, pyrazoline ring, piperidine ring, piperazine ring and morpholine ring among the above "5- or 6-membered heterocycle". Aromatic heterocycle is preferable.

The examples of "5- or 6-membered heterocycle" for Y, Y¹ and Y² are 4-pyridyl, 2-furyl, 3-furyl, 2-thienyl, 3-thienyl, 1,2-dihydropyridin-5-yl, 2,3-dihydropyridazin-6-yl and 1,2-dihydropyradin-5-yl.

Examples of "5- or 6-membered ring which may fuse with benzene ring" are indole ring, isoindole ring, benzimidazole ring, indazole ring, cinnoline ring, phthalazine ring, quinazoline ring, benzisoxazole ring, benzoxazole ring, benzoxadiazole ring, benzothiazole ring, benzisothiazole ring, benzofuran ring, benzothiophene ring, benzotriazole ring, isobenzofuran ring, chromene ring, indoline ring and isoindoline ring.

Substituents for "optionally substituted 5- or 6-membered heterocycle" and "optionally substituted 5- or 6-membered heterocycle which may fuse with benzene ring" are exemplified by
(i) halogen; (ii) hydroxy; (iii) lower alkyl optionally substituted with hydroxy or acyloxy; (iv) lower alkoxy optionally substituted with halogen, aryl or 5- or 6-membered heterocycle; (v) lower alkenyl; (vi) lower alkenyloxy; (vii) lower alkynyl; (viii) lower alkynyloxy; (ix) acyloxy; (x) carboxy; (xi) lower alkoxycarbonyl; (xii) mercapto; (xiii) lower alkylthio; (xiv) lower alkenylthio;
(xv) amino which may be mono- or di-substituted with
   halogen,
   lower alkyl optionally substituted with cycloalkyl or 5- or 6-membered heterocycle,
   acyl optionally substituted with halogen,
   lower alkenyl,
   cycloalkyl, or
   lower alkylsulfonyl;
(xvi) imino optionally substituted with lower alkylsulfonyl;
(xvii) hydrazino optionally substituted with
   lower alkyl,
   lower alkenyl,
   optionally substituted lower alkylidene or
   cycloalkylidene;
(xviii) aminooxy optionally substituted with
   lower alkyl,
   lower alkenyl,
   optionally substituted lower alkylidene or
   cycloalkylidene;
(xix) nitro; (xx) lower alkylsulfonyl; (xxi) aryl; (xxii) 5- or 6-membered heterocycle; (xxiii) oxo; and (xxiv) oxide.
These substituents may substitute at one or more of any possible positions.

The substituents for "optionally substituted 5- or 6-membered heterocycle which contains one or two of hetero atoms" are the same as the above. 5- or 6-membered heterocycle substituted with lower alkyl or unsubstituted one is preferable.

The terms "W¹, W² and/or W³ represents a single bond when ring A, ring B and/or ring C is optionally substituted 5-membered heterocycle" and "W³ represents a single bond when ring C is 5-membered heterocycle" mean as follows:
W¹ represents a single bond when ring A is 5-membered heterocycle, resulting in the binding positions of V¹ and X to ring A as shown below. 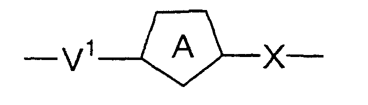
Each of W² and W³ represents a single bond when ring B or ring C is 5-membered heterocycle, resulting in the binding positions of V¹ and V² shown below. 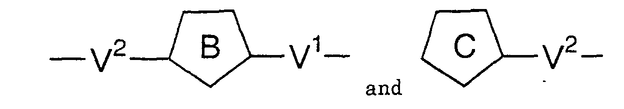
Each of X, V¹ and V² may directly bind to a hetero atom constituting ring A, ring B or ring C.

The phrase "R^{a} and R^{b} taken together may form -(CR^{e}R^{f})s-" means that R^{a} and R^{b} are taken together with a nitrogen atom to which R^{a} and R^{b} are attached may form optionally substituted nitrogen-containing saturated heterocycle, including, for example, optionally substituted aziridine, optionally substituted azetidine, optionally substituted pyrrolidine, optionally substituted piperidine and optionally substituted perhydroazepine (wherein the substituents are lower alkyl, lower alkoxy or amino). Each of plural R^{e} and plural R^{f} is independently e.g., hydrogen, lower alkyl, lower alkoxy or amino. R^{a} and R^{b} may form, for example, -(CH₂)₂-, -(CH₂)₃-, -CH(Me)(CH₂)₃-, -CH₂CH(OMe)(CH₂)₃- or -(CH₂)₃CH(NH₂)(CH₂)₂-.

As the pharmaceutically acceptable salt of the present compound (I), exemplified are salts with mineral acids such as hydrochloric acid, sulfuric acid, nitric acid, phosphoric acid, hydrofluoric acid and hydrobromic acid; salts with organic acids such as formic acid, acetic acid, tartaric acid, lactic acid, citric acid, fumaric acid, maleic acid and succinic acid; salts with organic base such as ammonium, trimethylammonium and triethylammonium; salts with alkaline metals such as sodium and potassium; and salts with alkaline earth metals such as calcium and magnesium.

Compound (I) includes solvate, preferably hydrates, and also includes all stereoisomers (for example, atropisomers etc.) thereof.

Compound (I) can be synthesized by the methods described in WO97/39999, WO98/04508, WO99/38829 and the like. Furthermore, Compound (I) can be synthesized by the following methods.

### Method A

Compound (Ic) can be produced from the compound represented by formula (II) (hereinafter referred to as Compound (II)) and the compound represented by formula (III) (hereinafter referred to as Compound (III)). 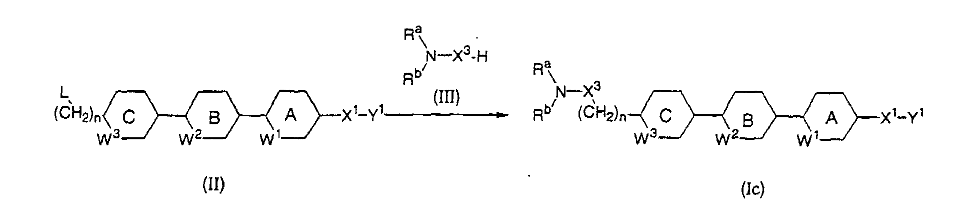 wherein substituent L is leaving group such as halogen, lower alkylsulfonyl, arylsulfonyl, lower alkylsulfonyloxy or arylsulfonyloxy and the other symbols are defined as described above.

Compounds (II) and (III) are reacted in a suitable solvent (for example, benzene, toluene, acetone, acetonitrile, tetrahydrofuran, N, N-dimethylformamide, dimethylsulfoxide, pyridine, methanol or ethanol) optionally in the presence of a base (for example, sodium hydride, potassium t-butoxide, pyridine, triethylamine, potassium carbonate, sodium hydroxide or potassium hydroxide) at 0 °C or with heating for several minutes to several tens hours to give intended Compound (Ic).

Compound (Ic') wherein R^{a} and R^{b} are taken together to form R^{c}R^{d}C= can be prepared by reacting Compound (Ic) wherein R^{a} and R^{b} are hydrogens obtained by Compound (II) with a compound of the formula (IV) (hereinafter referred to as Compound (IV)). 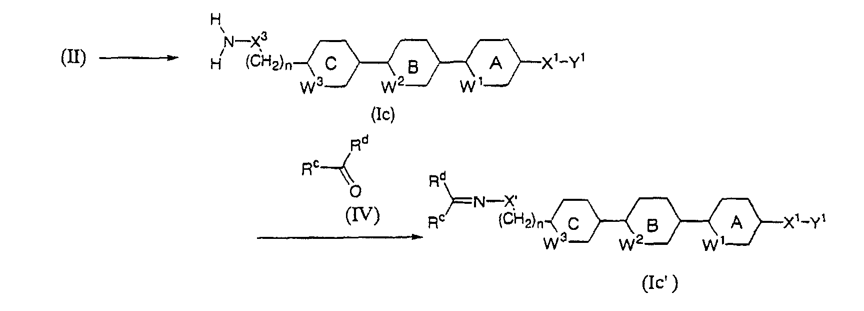 wherein each symbol is defined as described above.

First, Compound (II) is reacted with hydrazine₌in a suitable solvent (for example, toluene, tetrahydrofuran, N, N-dimethylformamide, dimethylsulfoxide, pyridine, methanol or ethanol) or without using any solvent to give Compound (Ic, X³=NH) or subjected to a method employing N-hydroxyphthalimide described in Journal of Chemical Society, 1926, 2348 or a method employing benzohydroxamic acid as described in Journal of Chemical Society, 1927, 874 to give Compound (Ic', X³=O). A compound thus obtained is subjected to a dehydration condensation with a carbonyl compound (IV) such as ketone or aldehyde optionally in the presence of an acid catalyst (hydrochloric acid, acetic acid, trifluoroacetic acid, lower alkanesulfonic acid, arylsulfonic acid and the like) to give intended Compound (Ic').

When one of R^{c} and R^{d} in Compound (Ic') is lower alkoxy, a reaction with a carbonyl compound (R^{c}C(=O)R^{d}) in a suitable solvent (for example, toluene, tetrahydrofuran, dioxane or dichloromethane) in the presence of an acid (hydrochloric acid, acetic acid, perchloric acid and the like) is effected, whereby converting into an intended compound having other R^{c} and R^{d}.

Compound (Ic) wherein R^{a} and R^{b} are hydrogens can also be synthesized from a compound represented by formula (II') (hereinafter referred to as Compound (II')): 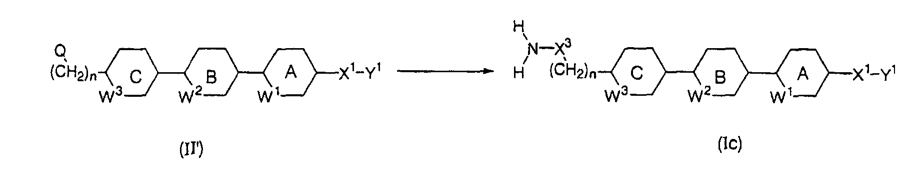 wherein Q is OH or amino and other symbols are defined as described above.

For example, Compound (II'; Q=OH) is converted into an alkoxide or phenoxide using a suitable base (for example, sodium hydride, potassium t-butoxide, potassium carbonate, sodium hydroxide and potassium hydroxide) and then reacted with chloramine or O-arylsulfonylhydroxylamine (Journal of Organic Chemistry, 1973 (38) 1239-1241) or Compound (II'; Q=NH₂) is reacted with chloramine or hydroxylamine-O-sulfonic acid (Journal of Organic Chemistry, 1949 (14) 813).

### Method B

Compound (Ic) can be produced also by reacting a compound represented by formula (V) (hereinafter referred to as Compound (V)) with a compound represented by formula (VI) (hereinafter referred to as Compound (VI)): 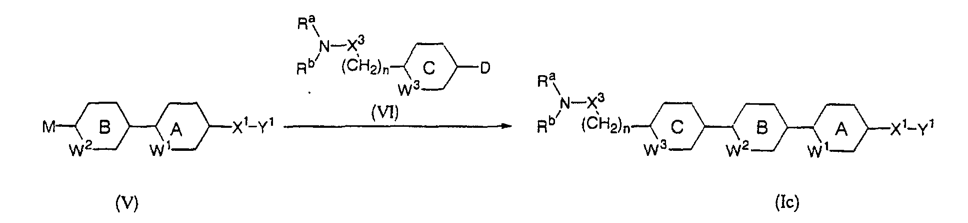 wherein one of M and D is dihydroxyboryl, di-lower alkylboryl or di-lower alkoxyboryl and the other is halogen or -OSO₂(C_{q}F_{2q+1}) wherein q is an integer of 0 to 4, and other symbols are defined as described above.

Compound (V) and Compound (VI) are reacted in a mixture of a suitable solvent (for example, benzene, toluene, N, N-dimethylformamide, dimethoxyethane, tetrahydrofuran, dioxane, ethanol or methanol) with water or in an anhydrous system in the presence of a palladium catalyst (for example Pd(PPh₃)₄, PdCl₂(PPh₃)₂, PdCl₂, Pd(OAc)₂ or PdCl₂(CH₃CN)₂, preferably Pd(PPh₃)₄) in a basic condition (with a base such as K₃PO₄, NaHCO₃, NaOEt, Na₂CO₃, Et₃N, Ba(OH)₂, Cs₂CO₃, CsF, NaOH or Ag₂CO₃) at room temperature or with heating for several tens minutes to several tens hours to give Compound (Ic).

Compound (VI) may be a known compound or may be obtained by a similar method for Compounds (Ic) and (Ic') described above.

One of substituent M and substituent D is any boryl group capable of being used in Suzuki reaction, and preferably dihydroxyboryl. The other is any leaving group applicable to Suzuki reaction, such as halogen or -OSO₂(C_{q}F_{2q+1}) wherein q is an integer of 0 to 4. Those preferred especially are halogen and trifluoromethanesulfonyloxy (hereinafter abbreviated as OTf), with bromine, iodine and OTf being most preferred.

Other substituents on ring A, ring B and ring C in Compounds (V) and (VI) and -X-Y are any groups by which Suzuki reaction is not affected adversely, including those except for halogen and -OSO₂(C_{q}F_{2q+1}) wherein q is an integer of 0 to 4, and even when any of the substituents on rings A, B and C is halogen, the reaction described above can satisfactorily be advanced when the reactivity between substituents M and D is higher relatively.

Compounds (II) and (II') in the reaction scheme shown above may be known compounds or can be synthesized by a known method or by the following method: 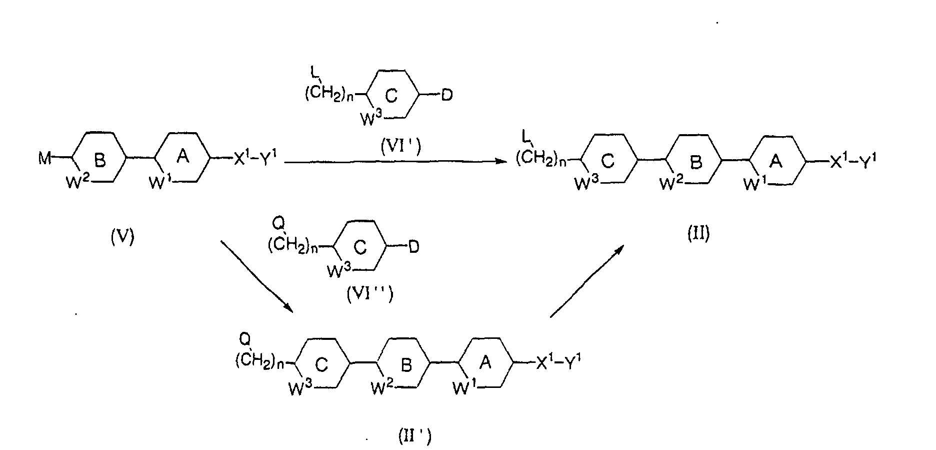 wherein substituent Q is NH₂ or a group by which Suzuki reaction is not affected adversely and which can be converted into substituent L by a standard method, and other symbols are defined as described above.

Using Compound (V) which is a known compound or obtained by a method described in WO98/04508 and Compound (VI') which is a known compound or obtained from a known compound by a standard method, Compound (II) is obtained by Suzuki reaction similarly to the steps described above. When substituent L has any adverse effect on Suzuki reaction, Compound (VI") is employed first to obtain Compound (II') and then substituent Q is converted into substituent L.

For example, when substituent Q is hydroxy, conversion into halogen can be accomplished under a standard condition, or, an intended compound can be obtained using a suitable sulfonylating agent (for example, methanesulfonyl chloride, p-toluenesulfonyl chloride or trifluoromethanesulfonic anhydride). Also when substituent Q is hydroxy which has previously been protected with a suitable protective group such as benzyl, t-butyldimethylsilyl or methoxymethyl, it is deprotected by a standard method into hydroxy, and subsequent procedure in accordance with the method described above results in an intended compound.

When substituent Q is lower alkylthio or optionally substituted arylthio, each may be converted into a corresponding sulfone form using a suitable oxidizing agent (for example, hydrogen peroxide, peracetic acid, m-chloroperbenzoic acid, oxone monopersulfate compound).

Compound (V) employed in the above reaction may be a known compound.

While Compound (I) is synthesized most efficiently and conveniently by Suzuki reaction described above, silicon, zinc or tin may also be employed instead of a boryl group in the scheme shown above as described in WO98/04508.

In the case of a compound having a substituent which interferes with a reaction described above, such group is protected first with a suitable protective group, which is then cleaved at an appropriate stage by a standard method. For example, when hydroxy interferes with a reaction, it is protected for example with methoxymethyl, methanesulfonyl, benzyl, trifluoromethanesulfonyl or t-butyldimethylsilyl, which is cleaved at an appropriate stage.

Compound (I) has suppressive effect of IgE production and differentiation from Th0 cells to Th2 cells, and so the oil preparation of the present invention containing Compound (I) as an active ingredient can be used as an immunosuppressant or anti-allergic agents. For example, a pharmaceutical composition containing the oil preparation of the present invention for use as an immunosuppressant or anti-allergic agents is useful for preventing or treating transplantation immunology (chronic GVHD), autoimmune diseases, atopic allergy diseases and the like.

The oil preparation of the present invention may contain 0.1 to 20 % by weight, preferably 0.5 to 20 % by weight, most preferably 1 to 10 % by weight of a high lipophilic compound as an active ingredient, each percentage being to the total amount of oil preparation. If the amount of the high lipophilic compound is more than the above range, the compound can not be perfectly dissolved in a basis and pharmacological effects of the composition are decreased. On the other hand, when the amount of the high lipophilic compound is less than the above range, the composition can not show sufficient pharmacological effects.

Examples of (i) MCT and/or PG for the oil preparation of the present invention are esters having 8 to 12 carbon atoms of various fatty acids.

Preferable examples of MCT are triglyceride with 2-ethylhexanoic acid, triglyceride with caprylic acid, triglyceride with capric acid, triglyceride with lauric acid and the mixture thereof. Concrete examples are commercially available MCT such as ODO (The Nisshin Oil Mills, Ltd.), Mygriol (Mitsuba Trading Co., Ltd.), Panacet (NOF Corporation), Trifat S-308 (Nikko Chemicals Co., Ltd.), Triestar₌ F-810 (Nikko Chemicals Co., Ltd.), Coconade (Kao Corporation), Myritol GM (Henkel Hakusui), T. C. G. (Kokyu Alcohol Kogyo Co., Ltd.), Sanfat MCT-6 (Taiyo Kagaku Co., Ltd.), Acter (Riken Vitamin Co., Ltd.). ODO (a mixture of the triglyceride tricaprylic acids ester and the triglyceride tricapric acids ester) or Mygriol is preferable and ODO is most preferable.

PG may be monoester, diester or a mixture thereof at an arbitrary ratio. Preferable examples are monoester of propylene glycol with caprylic acid, monoester of propylene glycol with capric acid, monoester of propylene glycol with lauric acid, diester of propylene glycol with caprylic acid, diester of propylene glycol with capric acid, diester of propylene glycol with lauric acid and the mixture thereof. Concrete examples are commercially available PG such as Sefsol 218 (Nikko Chemicals Co., Ltd.), Sefsol 228 (Nikko Chemicals Co., Ltd.), PDD (Nikko Chemicals Co., Ltd.), Mygriol 840 (Mitsuba Trading Co., Ltd.), Homotex (Kao Corporation). Sefsol 218 or Sefsol 228 is preferable.

MCT and PG can be used after mixing at an arbitrary ratio. Solo usage of MCT having 8 to 12 carbon atoms is preferable.
(ii) LCT for the oil preparation of the present invention is not limited and exemplified by vegetable oil such as sesame oil, corn oil, olive oil, soybean oil, avocado oil, grape seed oil, arachis oil, orange roughy oil, safflower oil, wheat germ oil, primrose oil, long chain fatty acid coconut oil, almond oil and apricot kernel oil, animal oil such as fish oil (cod-liver oil and the like) and mink oil, preferably vegetable oil, more preferably sesame oil, corn oil, olive oil or soy oil, and most preferably sesame oil or corn oil. These may be used those listed in The Japanese Pharmacopoea Twelfth Edition, The Pharmaceutical Additives Standard 1998, The Official Formulary of Food Additives Sixth Edition or the like.

The total amount of (i) MCT and/or PG and (ii) LCT is preferably 60 to 99 % by weight, more preferably 70 to 99 % by weight and most preferably 80 to 95 % by weight to total amount of the oil preparation. If the total amount is more than the above range, concentration of the active ingredient to the oil preparation is too lower to show sufficient pharmacological effects because of the decrease of the absorption volume. On the other hand, the total amount is less than the above range, a sufficient pharmacological effect can not be obtained because of decrease of oral absorbability.

A preferable weight ratio of (i) MCT and/or PG: (ii) LCT is in the range of 97:3 to 10:90. If LCT is more than the above range, the solubility of an active ingredient decreases though the oral absorbability increases. Additionally, since LCT is liquid or semisolid at room temperature, the oil preparation containing excess LCT is difficult to treat and causes problems such as turbidity or transformation of capsules during transportation. On the other hand, when the total amount of MCT and/or PG is more than the above range, oral absorbability decreases though solubility of an active ingredient increases. Therefore, sufficient pharmacological effects can not be obtained. In consideration of balance of oral absorbability and solubility, the weight ratio of (i) MCT and/or PG: (ii) LCT is preferably in a range of 95:5 to 10:90, more preferably 90:10 to 20:80, more preferably 70:30 to 30:70, more preferably 60:40 to 40:60 and most preferably 55:45 to 45:55.

The characteristic of the present preparation is concomitant use of (i) MCT and/or PG with (ii) LCT. Experiment 2 described later shows that BA by concomitant use of MCT with only a surfactant is not sufficient and further addition of LCT can remarkably increase oral absorbability.
(iii) Surfactant for the oil preparation of the present invention is not particularly limited and the examples are lecithin (soy lecithin, egg yolk lecithin and the like), hydrogenated lecithin, lysolecithin, synthetic phosphatidyl choline, polyglyceryl fatty acid ester (decaglyceryl trioleate, hexaglyceryl₌monooleate, tetraglyceryl monooleate, decaglyceryl monolaurylate and the like), alkylsulfate (sodium lauryl sulfate, sodium cetyl sulfate and the like), sucrose fatty acid ester, bile salt (sodium chorate, sodium deoxychorate and the like), sorbitan fatty acid ester (sorbitan sesquioleate, sorbitan trioleate, sorbitan monopalmitate and the like), sulfosuccinate ester (sodium dioctylsulfosuccinate and the like), glyceride (glyceryl monostearate and the like), polyoxyethyleneglycol alkyl ether (setomacrogol 1000, polyoxyethylenelaurylate ether, polyoxyethylenecetyl ether and the like), polyethyleneglycol fatty acid ester (polyethyleneglycol monostearate, polyethyleneglycol stearate ester and the like), polyoxyl stearate, polyoxyethyleneglycolated hydrogenated vegetable oil (polyoxyethylene (10) hydrogenated castor oil, polyoxyethylene (40) hydrogenated castor oil, polyoxyethylene (50) hydrogenated castor oil, polyoxyethylene (60) hydrogenated castor oil and the like), polyoxyethylene glycolated sorbitan fatty acid ester (polyoxyetbylene sorbitan monopalmitate (Tween 40), polyoxyethylene sorbitan monostearate (Tween 60), polyoxyethylene sorbitan tristearate (Tween 65), polyoxyethylene sorbitan monooleate (Tween 80), polyoxyethylene sorbitan monolaurate (Tween 20) and the like), polyoxyethyleneglycolated polypropylene glycol (Pluronic F87, Pluronic F68, Pluronic P123, Pluronic P85, Pluronic P84, Pluronic F127, Pluronic L-44 and the like) and polyethyleneglycol alkyl ether (polyoxyethylene lauryl ether and the like). Commercially available surfactants such as lecithin (Beisis (The Nisshin Oil Mills, Ltd.), SLP-White (True Lecithin MFG. Co., Ltd.)), Decaglyn (Nikko Chemicals Co., Ltd.), HCO=60 (Nikko Chemicals Co., Ltd.), Leodole TW-0120 (Kao Corporation) are preferable and soy lecithin is more preferable.

Surfactants may be contained 0.1 to 20 % by weight, preferably 0.5 to 15 % by weight and most preferably 1 to 10 % by weight to the total amount of the oil preparation. If the amount of a surfactant is more than the above range, pharmaceutical manufacturing is difficult because the surfactant can not be perfectly dissolved and the preparation is separated. If the amount of a surfactant is less than the above range, improvement of oral absorbability is not enough and the pharmaceutical effect can not be sufficiently obtained.

The oil preparation of the present invention may contain further additives, if necessary, an antiseptics such as methylparaben and butylparaben, a chelating agent such as sodium EDTA, an absorption enhancer such as capric acid, oleic acid, linolenic acid, arachidonic acid, 5-methoxysalicylic acid, azon, octylglucopyranoside, sodium salicylate, glycochorate sodium, sodium EDTA, sodium caprate, sodium deoxychorate, lauryl maltoside, polycarboxylic acid and oxycarboxylic acid, an antioxidant such as α-tocopherol, butylhydroxytoluene, butylhydroxyanisole, lipophilic vitamin C, L-cysteine, L-arginine and sodium thiosulfate and a lipophilic solubilizing agent such as triethyl citrate and triacetin.

The method for preparing pharmaceutical compositions using the oil preparation of the present invention is not limited. For example, after (ii) LCT and (iii) a surfactant are added to (i) MCT and/or PG and mixed, a high lipophilic compound as an active ingredient is dissolved therein. Using thus obtained mixture, a suitable form of a pharmaceutical may be prepared by a standard method. Preferable pharmaceutical forms are soft capsules and liquids, and more preferable forms are soft capsules.

The dosage of the oil preparation of the present invention should be determined taking into account the patient's age, body weight, and the type and degree of diseases. For example, a preparation which can administer the active ingredient in an amount of 0.1 - 500 mg/day, preferably 1 to 200 mg/day may be administered to an adult. The preparation may be administered once a day or in several parts per a day.

Since an usual preparation, such as tablets, granules and liquids (for example, ordinary solutions), containing lipophilic compound as an active ingredient is highly influenced by meals, BA after-meals-administration tends to be extremely different from one under-fasting-administration. But the oil preparation of the present invention continuously shows high AUC because of the dissolubility improvement of an active ingredient in gastrointestinal tract whereby accomplishing good oral absorbability even under fasting. Furthermore, the present preparation scarcely causes side effects, because the preparation can show enough efficacy at low dosage by improvement of oral absorbability.

### Examples

The present invention is further explained by the following Examples and Experiments, which are not intended to limit the scope of the present invention.

### Example-1

### Experimental method

(1) Compound (I-1) (a compound of the formula (Ia) wherein each of R⁴, R⁵, R⁶, R⁷, R¹², R¹⁴ and R¹⁵ is H, each of R⁸, R⁹, R¹⁰ and R¹¹ is methyl, R¹³ is F, X¹ is NH, Y¹ is prenyl, X² is O, and Y² is prenyl) was used as an active ingredient. The present preparation was prepared by dissolving of 50 mg/g (5%) of an active ingredient in oily vehicle, composed of 5% soybean lecithin, 45% Miglyol 812 and 45% sesame oil. The reference preparation was prepared by suspending of 5 mg/mL (0.5%) of an active ingredient in methyl cellulose aqueous solution(0.5% MC / 99% water).
(2) The present preparation prepared as above was administered orally to fasted or non-fasted beagle dogs (about 15-month age, male, about 11 Kg body weight) at a dose of 1g/dog. 2mL of blood samples were taken from forelimb vein at 1, 2, 4, 6, 8, 10 and 24h after the administration. The blood was centrifuged and the concentration of active ingredient in plasma was analyzed by HPLC. AUC in plasma was calculated by trapezoidal rule. The reference preparation was administered orally to beagle dogs at a dose of 10mL/dog. (Dose: 50mg/dog≒4.5mg/Kg, n=3)

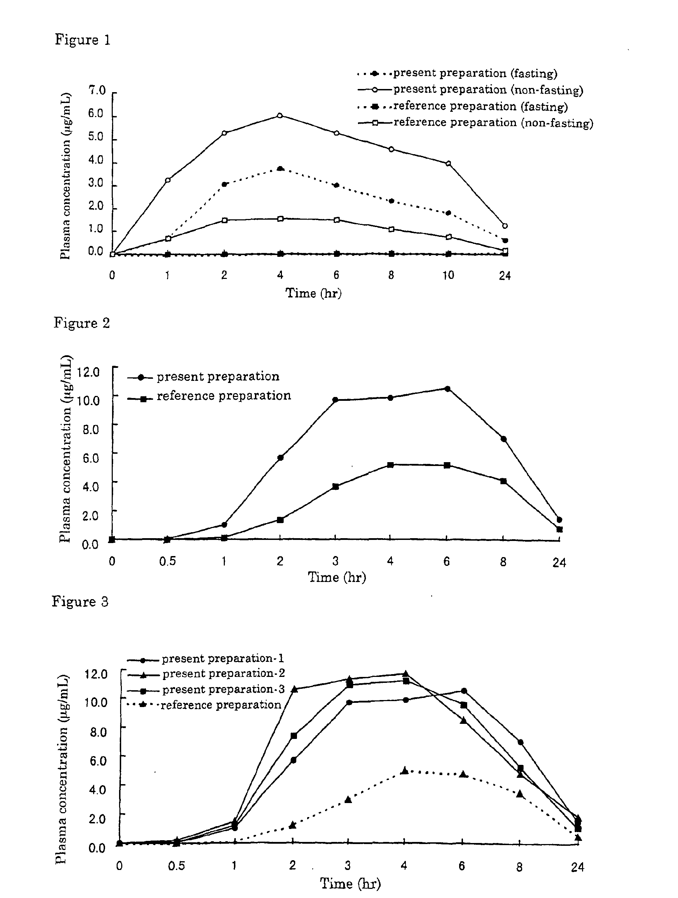

The results are shown in Fig.1 and Table.1.

**Table.1**

| preparations | conditions | AUC (µg*hr/mL) | Cmax. (µg/mL) | AUC ratio (non-fasting/fasting) |
|---|---|---|---|---|
| present preparation | fasting | 25.29 | 3.79 | 158.1 |
| | non-fasting | 46.91 | 6.04 | 293.2(1.85) |
| reference preparation | fasting | 0.16 | 0.03 | 1.0 |
| | non-fasting | 14.79 | 2.24 | 92.4(92.4) |

### Example-2

### Experimental method

(1) Compound (I-2) (a compound of the formula (Ib) wherein each of R⁴, R⁶, R⁷, R⁸ and R¹¹ is H, R⁵ is F, each of R⁹and R¹⁰ is methyl, ring C is 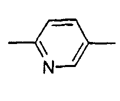 X¹ is NH, Y¹ is prenyl, X² is O, and Y² is prenyl) was used as an active ingredient. The present preparation was prepared by dissolving 10 mg/g (1%) of an active ingredient in oily vehicle, composed of 5% soybean lecithin, 47% ODO and 47% sesame oil. The reference preparation was prepared by dissolving 10 mg/g (1%) of an active ingredient in oily vehicle, composed of 5% soybean lecithin and 94% ODO.
(2) The present preparation or reference preparation prepared as above were administered orally to fasted rats (Jcl-SD strain, male, about 8-weeks age, about 300g body weight) at a dose of 0.6 mL/rat. (The dose of the active ingredient was 20mg/Kg, n=3) Blood samples (0.25 ml) were taken from jugular vein at 0.5, 1, 2, 3, 4, 6, 8 and 24h after the administration. The blood was centrifuged and the concentration of active ingredient in plasma was analyzed by HPLC. AUC in plasma was calculated by trapezoidal rule. The results are shown in Fig.2 and Table.2.

**Table.2**

| preparations | conditions | AUC(µg*hr/mL) | Cmax. (µg/mL) | AUC ratio |
|---|---|---|---|---|
| present preparation | fasting | 127.4 | 11.04 | 1.00 |
| reference preparation | fasting | 66.7 | 5.50 | 0.52 |

### Example-3

### Experimental method

(1) The present preparation was prepared by dissolving 10 m/g (1%) of compound (1-2) as an active ingredient in oily vehicle, composed with 5% soybean lecithin, 47% Miglyol 812 and 47% sesame oil (or corn, olive oil). The reference preparation was prepared by dissolving 10 mg/g (1%) of compound (I-2) in oily vehicle, composed with 5% soybean lecithin and 94% Miglyol 812.
(2) The present preparation or the reference preparation prepared as above were administered orally to fasted rats (Jcl-SD strain, male, about 8-weeks age, about 300g body weight) at a dose of 0.6 mL/rat. (The dose of the active ingredient was 20mg/Kg, n=3) 0.25 mL of blood samples were taken from jugular vein at 0.5, 1, 2, 3, 4, 6, 8 and 24h after the administration. The blood was centrifuged and the concentration of the active ingredient in plasma was analyzed by HPLC. AUC in plasma was calculated by trapezoidal rule.

The results are shown in Fig.3 and Table.3.

**Table.3**

| preparations | oil ingredients | conditions | AUC (µg*hr/mL) | Cmax. (µg/mL) | AUC ratio |
|---|---|---|---|---|---|
| present preparation-1 | sesame oil | fasting | 127.4 | 11.04 | 2.24 |
| present preparation-2 | corn oil | fasting | 115.8 | 11.71 | 2.04 |
| present preparation-3 | olive oil | fasting | 111.1 | 11.61 | 1.95 |
| reference preparation | non | fasting | 56.9 | 5.10 | 1 |

### Example-4

### Experimental method

(1) Compound (I-1) was used as the active ingredient. The present preparation was prepared by dissolving 50 mg/g (5%) of compound (I-1) in oily vehicle, composed with 5% soybean lecithin and 90 % the mixture oil (corn oil and ODO). The reference preparation-1 was prepared by dissolving 50 mg/mL of compound (I-1) in ODO oil. The reference preparation-2 was prepared by dissolving 50 mg/g of compound (I-1) in corn oil.
(2) The present preparation and reference preparations prepared as above were administered orally to fasted rats (Jcl-SD strain, male, about 8-weeks age, about 300g body weight) at a dose of 0.3 mL/rat. (Dose of active ingredient:50mg/Kg, n=3) 0.25 mL of blood samples were taken from jugular vein at 0.5, 1, 2, 3, 4, 6, 8 and 24h after the administration. The blood was centrifuged and the concentration of active ingredient in plasma was analyzed by HPLC. The AUC in plasma was calculated by trapezoidal rule. AUC ratio and Cmax ratio were calculated by comparing to the AUC and Cmax values in reference praparation-1 which is assumed as 1.00.

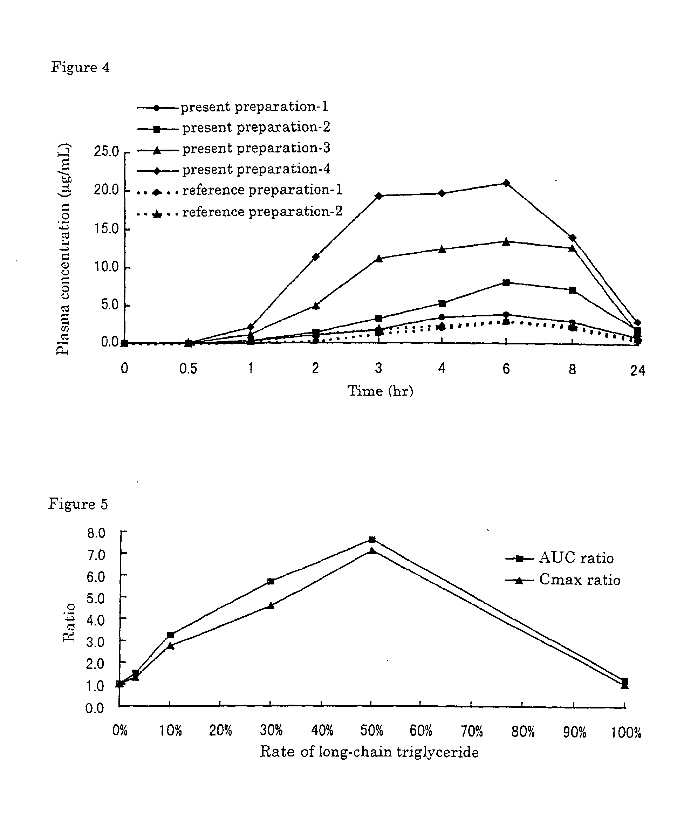

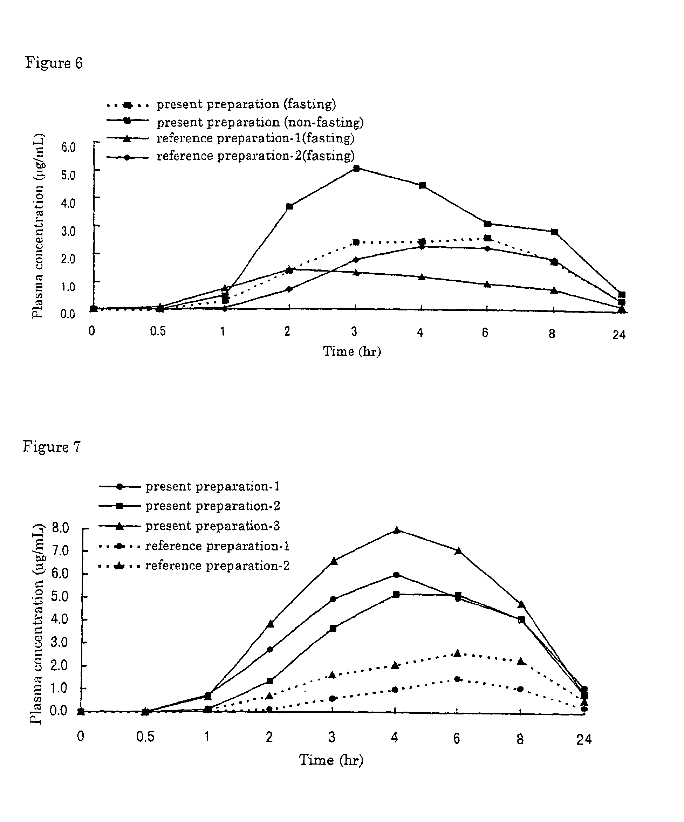

The results are shown in Table.4 and Fig.4 and 5.

**Table.4**

| preparations | conditions | corn oil concentration (corn oil/ODO) | AUC | | Cmax | |
|---|---|---|---|---|---|---|
| | | | (µg*hr/mL) | ratio | (µg/mL) | ratio |
| reference preparation-1 | fasting | 0% | 33.5 | 1.00 | 2.96 | 1.00 |
| present preparation-1 | fasting | 3% | 49.7 | 1.48 | 3.86 | 1.30 |
| present preparation-2 | fasting | 10% | 109.3 | 3.26 | 8.10 | 2.74 |
| present preparation-3 | fasting | 30% | 190.2 | 5.68 | 13.52 | 4.56 |
| present preparation-4 | fasting | 50% | 254.7 | 7.60 | 21.04 | 7.11 |
| reference preparation-2 | fasting | 100% | 40.6 | 1.21 | 2.97 | 1.00 |

### Example-5

### Experimental method

(1) Compound (I-2) was used as the active ingredient. The present preparation was prepared by dissolving 10 mg/g (1%) of the active ingredient in oil vehicle, composed of 5% soybean lecithin, 47% sesame oil and 47% ODO. The reference preparation-1 was prepared by suspending 10 mg/mL (1%) of the active ingredient in methyl cellulose aqueous solution (98.5% water/0.5% MC) and the reference preparation-2 was also prepared by dissolving 10 mg/mL of the active ingredient in ODO.
(2) The present preparation and two reference preparations prepared as above were administered orally to fasted or non-fasted rats (Jcl-SD strain, male, about 8-weeks age, about 300g body weight) at a dose of 0.3 mL/rat. (Dose of the active ingredient: 10mg/Kg, n=3) 0.25 mL of blood samples were taken from jugular vein at 0.5, 1, 2, 3, 4, 6, 8 and 24h after the administration. The blood was centrifuged and the concentration of active ingredient in plasma was analyzed by HPLC. The AUC value in plasma was calculated by trapezoidal rule.

The results are shown in Table 5 and Fig. 6.

**Table.5**

| preparations | conditions | AUC (µg*hr/mL) | Cmax. (µg/mL) | AUC ratio (fasting /non-fasting) |
|---|---|---|---|---|
| present preparation-1 | fasting | 32.37 | 2.65 | 2.15 |
| | non-fasting | 53.53 | 5.10 | 3.56(0.60) |
| reference preparation-1 | non-fasting | 15.05 | 1.45 | 1 |
| reference preparation-2 | non-fasting | 29.94 | 2.30 | 1.99 |

### Example-6

### Experimental method

(1) Compound (I-1) was used as an active ingredient. The present preparation was prepared by dissolving 5 mg/g (0.5%) of the active ingredient in oil vehicle composed from 5% to 20% surfactants and 94.5% to 79.50% mixture oil of Panacet 810/sesami oil(1/1). The reference preparation-1 was prepared by dissolving 5 mg/g of the active ingredient in Panacet 810 and the reference preparation-2 dissolved 5 mg/g of the active ingredient in Panacet 810/sesami oil(1/1) mixture.
(2) The present preparation and reference preparations prepared as above were administered orally to fasted rats (Jcl-SD strain, male, about 8-weeks age, about 300g body weight) at a dose of 0.6 mL/rat. (Dose of the active ingredient: 10mg/Kg, n=3) 0.25 mL of blood samples were taken from jugular at 0.5, 1, 2, 3, 4, 6, 8 and 24h after the administration. The blood was centrifuged and the concentration of active ingredient in plasma was analyzed by HPLC. The AUC value was calculated by trapezoidal rule.

The results are shown in Table.6 and Fig.7.

**Table.6**

| preparations | conditions | surfactants | | AUC (µg*hr/mL) | Cmax. (µg/mL) | AUC ratio |
|---|---|---|---|---|---|---|
| | | ingredients | concentration | | | |
| present preparation-1 | fasting | decaglyn | 20% | 73.07 | 7.09 | 4.30 |
| present preparation-2 | fasting | Soybean lecithin | 5% | 66.71 | 5.50 | 3.92 |
| present preparation-3 | fasting | Soybean lecithin | 20% | 87.06 | 7.98 | 5.12 |
| reference preparation-1 | fasting | Non | | 17.00 | 1.50 | 1.00 |
| reference preparation-2 | fasting | Non | | 36.11 | 2.63 | 2.12 |

### Examples 1 to 6

The oil preparations are prepared as follows:
Mixed with the following ingredients, then Compound (I) is dissolved or suspended to prepare the present oil preparations.

| Example 1 | |
|---|---|
| Compound (I-1) | 10% |
| Miglyol 812 | 60% |
| Avocado oil | 10% |
| Sorbitan monopalmitate | 20% |

| Example 2 | |
|---|---|
| Compound (I-2) | 5% |
| Mylitol GM | 30% |
| Corn oil | 60% |
| Polyethyleneglycol monostearate | 5% |

| Example 3 | |
|---|---|
| Compound (I-1) | 12% |
| Panacet | 34% |
| Arachis oil | 34% |
| Polyoxyethylene(40) hydrogenated castor oil | 20% |

| Example 4 | |
|---|---|
| Compound (I-2) | 5% |
| Coconard | 65% |
| Safflower oil | 29% |
| Pluronic F87 | 1% |

| Example 5 | |
|---|---|
| Compound (I-1) | 0.5% |
| Acter | 87% |
| Mink oil | 3% |
| Hydrogenated lecithin | 9.5% |

| Example 6 | |
|---|---|
| Compound (I-2) | 5% |
| ODO | 45% |
| Olive oil | 45% |
| Soybean lecithin | 5% |

### Industrial Applicability

The pharmaceutical using the oil preparation of the present invention is useful for an oral pharmaceutical because it shows superior BA and the BA is not scarcely influenced by the kind of meals, with or without feeding, dietary intake and an individual difference.

## Claims

1. An oil preparation comprising a high lipophilic medicament as an active ingredient, (i) a medium-chain triglyceride and/or a propylene glycol medium-chain fatty acid ester, (ii) a long-chain triglyceride and (iii) a surfactant.

2. The oil preparation as claimed in claim 1, wherein the active ingredient is a compound of the formula (I): 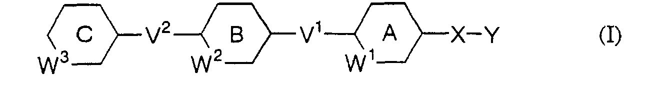 wherein the ring A, ring B and ring C are each independently optionally substituted aromatic carbocycle or optionally substituted 5- or 6-membered heterocycle which may fuse with benzene ring,
W¹, W² and/or W³ represents a single bond when the ring A, ring B and/or ring C is optionally substituted 5-membered heterocycle,
X is a single bond, -O-, -CH₂-, -NR¹- wherein R¹ is hydrogen, optionally substituted lower alkyl, lower alkenyl or lower alkylcarbonyl, or -S(O)p- wherein p is an integer of 0 to 2,
Y is hydrogen, optionally substituted lower alkyl, optionally substituted lower alkoxy, optionally substituted lower alkenyl, optionally substituted lower alkynyl, optionally substituted acyl, optionally substituted cycloalkyl, optionally substituted cycloalkenyl, optionally substituted lower alkoxycarbonyl, optionally substituted sulfamoyl, optionally substituted amino, optionally substituted aryl or optionally substituted 5- or 6-membered heterocycle,
R¹ taken together with Y may form -(CH₂)m-, -(CH₂)₂-Q-(CH₂)₂- (wherein Q is CH₂, O, S or NR'), -CR'=CH-CH=CR'-, -CH=N-CH=CH-, -N=CH-N=CH-, -C(=O)-O(CH₂)r-, -C(=O)-NR'-(CH₂)r- or -C(=O)-NR'-N=CH- wherein m is 4 or 5, r is 2 or 3, R' is hydrogen, lower alkyl or lower alkenyl,
Y may be halogen when X is -CH₂- or -NR¹-,
Y may be optionally substituted lower alkylsulfonyl or optionally substituted arylsulfonyl when X is -O- or -NR¹-,
one of V¹ and V² is a single bond and the other is a single bond, -O-, -NH-, -OCH₂-, -CH₂O-, -CH=CH-, -C=C-, -CH(OR²)- wherein R² is hydrogen or lower alkyl, -CO-, -NHCHR³- or -CH R³NH- wherein R³ is hydrogen or hydroxy,
or a pharmaceutically acceptable salt or solvate thereof as an active ingredient.

3. The oil preparation as claimed in claim 2, wherein (i) the medium-chain triglyceride and/or the propylene glycol medium-chain fatty acid ester is a mixture of triglyceride with caprylic acid and triglyceride with capric acid.

4. The oil preparation as claimed in any one of claims 1 to 3 wherein (ii) the long-chain triglyceride is vegetable oil.

5. The oil preparation as claimed in any one of claims 1 to 4 wherein (iii) the surfactant is lecithin.

6. The oil preparation as claimed in any one of claims 1 to 5 wherein the active ingredient is 0.1 to 20 % by weight, the total amount of (i) a medium-chain triglyceride and/or a propylene glycol medium-chain fatty acid ester and (ii) a long-chain triglyceride is 60 to 99 % by weight and (iii) a surfactant is 0.1 to 20 % by weight, each percentage being to the total amount of the oil preparation.

7. The oil preparation as claimed in any one of claims 1 to 5 wherein the active ingredient is 1 to 10 % by weight, the total amount of (i) a medium-chain triglycenide and/or a propylene glycol medium-chain fatty acid ester and (ii) a long-chain triglyceride is 80 to 95 % by weight and (iii) a surfactant is 1 to 10 % by weight, each percentage being to the total amount of the oil preparation.

8. The oil preparation as claimed in any one of claims 1 to 7, wherein the weight ratio of (i) a medium-chain triglyceride and/or a propylene glycol medium-chain fatty acid ester and (ii) a long-chain triglyceride is in a range of 97:3 to 10:90.

9. The oil preparation as claimed in any one of claims 1 to 7, wherein the weight ratio of (i) a medium-chain triglyceride and/or a propylene glycol medium-chain fatty acid ester and (ii) a long-chain triglyceride is in a range of 70:30 to 30:70.

10. The oil preparation as claimed in any one of claims 1 to 9 wherein the active ingredient is a compound of the formula (Ia): 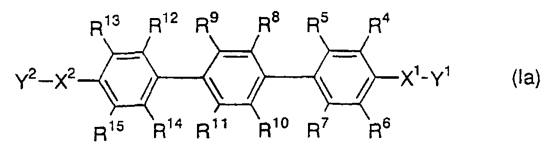 wherein R⁴, R⁵, R⁶, R⁷, R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, carboxy or lower alkoxycarbonyl,
X¹ and X² are each independently -O-, -CH₂- or -NH-,
Y¹ and Y² are each independently optionally substituted lower alkyl, optionally substituted aryl(lower)alkyl or optionally substituted lower alkenyl,
or a pharmaceutically acceptable salt or solvate thereof.

11. The oil preparation as claimed in any one of claims 1 to 9 wherein the active ingredient is a compound of the formula (Ib): 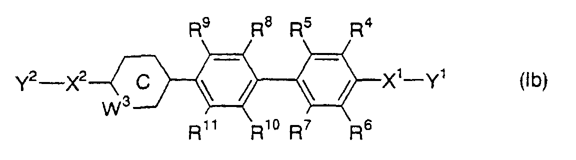 wherein the ring C is optionally substituted 5- or 6-membered heterocycle which contains one or two hetero atoms, W³ represents a single bond when the ring C is a 5-membered heterocycle and the other symbols are the same as defined in claim 9,
pharmaceutically acceptable salt or solvate thereof.

12. A pharmaceutical preparation comprising the oil preparation according to any one of claims 1 to 11.

13. The pharmaceutical preparation as claimed in claim 12 which is a soft capsule preparation.
